# EUROPÄISCHE PATENTSCHRIFT

(11) **EP 2 243 764 B1**
(45) Veröffentlichungstag und Bekanntmachung des Hinweises auf die Patenterteilung: **07.06.2017**
(21) Anmeldenummer: 10157058.8
(22) Anmeldetag: 19.03.2010
(51) Int. Cl.: C07C 45/46, C07C 50/28, C07C 50/30, C07C 46/06

(54) **Verfahren zur Herstellung von substituierten 1,4-Chinonmethiden**
Method for producing substituted 1.4 chinonmethides
Procédé de fabrication de 1,4-quinone méthides substitués

(30) Priorität: 21.04.2009 DE 102009002514
(43) Veröffentlichungstag der Anmeldung: 27.10.2010
(73) Patentinhaber: Evonik Degussa GmbH, 45128 Essen (DE)
(72) Erfinder: Rinker, Stefanie, Dr., 46569 Hünxe (DE); James, Phillip, R., Dr., Tenby SA70 7LR (GB); Neumann, Manfred, Dr., 45770 Marl (DE); Erpeldinger, Oliver, 42489 Wülfrath (DE); Kraushaar, Frank, Dr., 45239 Essen (DE)

(56) Entgegenhaltungen:
- EP-A1- 0 626 377
- WO-A1-2008/029370

## Beschreibung

Die vorliegende Erfindung beschreibt ein Verfahren zur Herstellung von substituierten 1,4-Chinonmethiden gemäß Formel **(I)** aus 3,5-disubstituierten 4-Hydroxybenzaldehyden, als auch ein Verfahren zur Herstellung der 3,5-disubstituierten 4-Hydroxybenzaldehyden aus den entsprechenden 2,6-disubstituierten Phenolen.

7-Methoxy- und 7-Ethoxy-substituierte 1,4-Chinonmethide sind als wichtige isolierbare Zwischenprodukte für die Synthese von pharmazeutischen Wirkstoffen in der Literatur bekannt. Ferner können einige 1,4-Chinonmethide zur Vermeidung von unerwünschter Polymerisation von olefinisch ungesättigten Monomeren eingesetzt werden.

Die Herstellung von 2,6-Di-*tert*.butyl-4-methoxymethylencyclohexan-2,5-dienon bzw. von 2,6-Di-*tert*.butyl-4-ethoxymethylencyclohexan-2,5-dienon beschreiben Inagaki et al. sowohl in J. Org. Chem. 2002, 67, 125-128 als auch in EP 0 626 377 A1. Hierbei wird eine Mischung aus 3,5-Di-*tert*.-butyl-4-hydroxybenzaldehyd mit einem Überschuss an Trimethylorthoformiat, absolutem Methanol und Xylol in Gegenwart von Ammoniumchlorid als Katalysator zum entsprechenden Acetal umgesetzt, indem die Reaktionsmischung für einige Stunden unter Rückfluss erhitzt wird. Anschließend wird eine Destillation durchgeführt, Xylol als zusätzliches Lösemittel zugefügt, abgekühlt und dann wird der Katalysator - das Ammoniumchlorid - abfiltriert. Um die Eliminierung des Alkohols aus dem Acetal zum substituierten 1,4-Chinonmethid zu erreichen, wird in beiden Publikationen das Filtrat erhitzt und somit Methanol und Xylol abdestilliert. Dabei konzentriert sich das Produkt auf, wird abfiltriert und anschließend in Hexan bzw. in einer Mischung aus Petrolether und Ligroin umkristallisiert.

Die Herstellung des entsprechenden Acetals des 3,5-disubstituierten 4-Hydroxybenzaldehyds durch Umsetzung mit Orthoformiat und/oder Alkoholen beschreiben zahlreiche Publikationen:
Orlando beschreibt in J. Org. Chem. 1970, 35, 3714-3717 ein fast identisches Verfahren zur Herstellung des Acetals wie Inagaki et al. in ihren beiden Publikationen. Auch hier wird 3,5-Di-*tert*.-butyl-4-hydroxybenzaldehyd mit einem Überschuss an Trimethylorthoformiat und absolutem Methanol in Gegenwart von Ammoniumchlorid als Katalysator unter Rückfluss erhitzt, wobei jedoch kein zusätzliches Lösemittel in diesem Verfahren eingesetzt wird. Das Acetal wird nach der Filtration durch Einengen und Umkristallisation aus Hexan isoliert.

Auch Roth et al. beschreiben in J. Med. Chem. 1988, 31, 122-129 ein Verfahren zur Herstellung des Acetals aus 3,5-disubstituierten 4-Hydroxybenzaldehyden, wobei auch hier eine Mischung aus 3,5-Diisopropyl-4-hydroxybenzaldehyd, Trimethylorthoformiat, Ammoniumchlorid und Methanol für einige Stunden unter Rückfluss erhitzt wird. Anschließend lässt man die Reaktionsmischung abkühlen, fügt eine wässrige Ammoniumhydroxid-Lösung hinzu, extrahiert mit Dichlormethan, wäscht und trocknet die organische Phase und engt diese bis zur Trockene ein. Das gewünschte Acetal lässt sich nun aus heißem Hexan auskristallisieren.

Die Herstellung von Acetalen anderer 4-Hydroxybenzaldehyde mit Trimethylorthoformiat und/oder Methanol in Gegenwart von verschiedenen Katalysatoren wird in zahlreichen Publikationen beschrieben. So beschreiben Du et al. in Synthetic Communications 2005, 35, 2703-2708 den Einsatz von ionischen Flüssigkeiten als Katalysator. Den Einsatz von Amidosulfonsäure als Katalysator beschreiben Gong et al. in Synthetic Communications 2004, 34, 4243-4247. Lithiumtetrafluoroborat als geeigneter Katalysator beschreiben Hamada et al. in Synlett 2004, 6, 1074-1076. Während Ranu et al. die Verwendung von Indiumchlorid als Katalysator in Adv. Synth. Catal. 2004, 346(4), 446-450 beschreiben. Gopinath et al. beschreiben in J. Org. Chem. 2002, 67, 5842-5845 ein Verfahren zur Herstellung des Acetals in Gegenwart von Tetrabutylammoniumchlorid als Katalysator. Den Einsatz des hoch-toxischen Decaborans als Katalysator beschreiben Lee et al. in Tetrahedron Letters 2002, 43, 2699-2703. Ein Copolymer mit Galliumtrichlorid als geeigneten Katalysator beschreiben Ruicheng et al. in J. Macromol. Sci.-Chem. 1987, A24(6), 669-679.

In der Literatur sind viele verschiedene Wege beschrieben, um 3,5-substituierte 4-Hydroxybenzaldehyde herzustellen. Hauptausgangsmaterialien sind hierbei die entsprechenden 2,6-disubstituierten Phenole oder 2,6-disubstituierte 4-Methylphenole. Eine Möglichkeit zur Herstellung dieser 3,5-substituierte 4-Hydroxybenzaldehyde ist die Formylierung der 2,6-disubstituierten Phenole in para-Position mit Urotropin.

WO 2008/029370 offenbart die Umsetzung 2-Ethyl-6-methylphenol und Urotropin in Essigsäure und Wasser.

So beschreiben Bolli et al. in den beiden PCT-Veröffentlichungen WO 2006/100633 A1 und WO 2006/010544 A2 die Umsetzung von 2-Ethyl-6-methylphenol bzw. 2,6-Diethylphenol mit einem Überschuss an Urotropin in Gegenwart von Essigsäure. Die Reaktionsmischung wird nach Abdestillieren einer ersten Lösemittelfraktion für drei Stunden unter Rückfluss erhitzt, mit Wasser verdünnt und anschließend das entsprechende 4-Hydroxybenzaldehyd mit Ethylacetat extrahiert. Die Ausbeuten liegen bei 31 % bzw. 40 %.

Unangst et al. beschreiben in J. Med. Chem. 1994, 37, 322-328 die Umsetzung von 3,5-Diphenylphenol mit einem Überschuss an Urotropin in Gegenwart von Essigsäure. Hierbei wird Wasser zugesetzt, das Reaktionsgemisch unter Rückfluss erhitzt und Destillat abgenommen bis eine Temperatur von 114 °C erzielt wird. Die Ausbeute liegt bei 64 %.

Ein Verfahren mit einer Ausbeute von 81 % beschreiben Roth et al. in J. Med. Chem. 1988, 31, 122-129. Hier wird 3,5-Diisopropylphenol mit einem Überschuss an Urotropin in Gegenwart von Eisessig und Wasser umgesetzt, wobei auch hier zunächst Destillat abgenommen wird, bevor die Reaktionsmischung unter Rückfluss erhitzt wird. Aufgabe der vorliegenden Erfindung war es, ein Verfahren zur Herstellung von substituierten 1,4-Chinonmethiden der Formel **(I)** zur Verfügung zu stellen, das geeignet ist, großtechnisch angewandt zu werden und somit wirtschaftlich und anlagenschonend ist. Zudem sollten nicht nur die in der Literatur beschriebenen 7-Methoxy- und 7-Ethoxy-substituierten 1,4-Chinonmethide herstellbar sein, sondern auch weitere substituierte 1,4-Chinonmethide gemäß der Formel **(I).**

Überraschenderweise wurde ein breit anwendbares Verfahren zur Herstellung von substituierten 1,4-Chinonmethiden gemäß der Formel **(I)** gefunden, welches sich dadurch auszeichnet, dass zunächst ein (Thio-)Acetal ausgehend von 4-Hydroxybenzaldehyden der Formel **(II)** gebildet wird, welches in einem weiteren Schritt durch thermisch induzierte Eliminierung von Alkohol oder Thiol direkt zum gewünschten substituierten 1,4-Chinonmethiden umgesetzt werden kann. Mit diesem erfindungsgemäßen Verfahren sind nicht nur die in der Literatur beschriebenen 7-Methoxy- und 7-Ethoxy-substituierten 1,4-Chinonmethide herstellbar, sondern auch völlig neue Verbindungen zugänglich.

Im erfindungsgemäßen Verfahren können zur Bildung der (Thio-)Acetale im Gegensatz zum Stand der Technik preiswerte, ungiftige und halogenfreie Katalysatoren wie zum Beispiel organische Sulfonsäuren, Schwefelsäure und/oder deren Hydrogensulfate als Katalysator eingesetzt werden. Somit ist eine halogenfreie Herstellung möglich. Gerade bei großtechnischen Verfahren ist dies auf Grund der Gefahr von Spannungsrisskorrosion beim Einsatz von Halogeniden im Reaktor ein wichtiger Vorteil. Völlig überraschend war es, dass auch preiswerte, untoxische Substanzen, die Acetalbildung katalysieren, eingesetzt werden können, wohingegen der Stand der Technik außer halogenhaltigen Verbindungen, nur teure, toxische und/oder CMR-aktive Verbindungen, wie das Decaboran, als Katalysator vorschlägt.

Ferner kann der Anteil an dem Katalysator gegenüber Verfahren gemäß dem Stand der Technik herabgesenkt werden, wobei überraschenderweise der Umsatz leicht ansteigt und nicht wie eventuell erwartet absinkt. Zudem kann im Gegensatz zu vielen Verfahren des Stands der Technik der Anteil an dem teuren Edukt Orthoformiat deutlich abgesenkt werden, wobei gleichbleibende Umsätze von über 90 % erreicht werden. Da das erfindungsgemäße Verfahren in der ersten Verfahrenstufe ohne ein zusätzliches Lösemittel auskommen kann, kann zudem die Raum-Zeit-Ausbeute deutlich verbessert werden.

Bei dem erfindungsgemäßen Verfahren werden 3,5-disubstituierte 4-Hydroxybenzaldehyde der Formel **(II)** als Edukt eingesetzt, im Rahmen dieser Erfindung konnte auch der vorgeschaltene Verfahrensschritt die Herstellung des 3,5-disubstituierte 4-Hydroxybenzaldehyds aus dem 2,6-disubstiutierten Phenol verbessert werden. So zeigte sich völlig überraschend, dass sowohl durch die Änderung der Dosierreihenfolge als auch durch den Einsatz einer Reaktionstemperatur unterhalb der Rückflusstemperatur Ausbeuten von über 80 % erzielt werden können. Ferner war es überraschend, dass die molare Menge an Urotropin bezogen auf das eingesetzte 2,6-disubstiutierten Phenol auf unter 1 : 1 gesenkt werden kann, ohne Einbußen in der Ausbeute hinnehmen zu müssen.

Gegenstand der Erfindung ist somit ein Verfahren zur Herstellung von substituierten 1,4-Chinonmethiden der Formel **(I)** 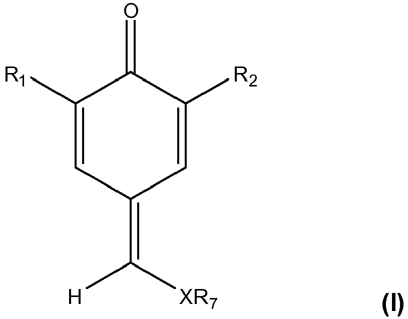 mit
- R₁, R₂ =: unabhängig voneinander Wasserstoff, (C₁-C₁₅)-Alkyl, (C₃-C₁₅)-Cycloalkyl oder (C₆-C₁₄)-Aryl, wobei diese substituiert oder unsubstituiert sind,
- R₇ =: (C₁-C₁₅)-Alkyl, (C₃-C₁₅)-Cycloalkyl oder (C₆-C₁₄)-Aryl, wobei diese substituiert oder unsubstituiert sind, und
- X =: O, S,
das dadurch gekennzeichnet ist, dass 3,5-disubstituierte 4-Hydroxybenzaldehyde der Formel **(II)** 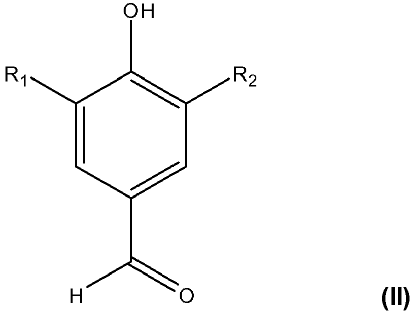 wobei R₁ und R₂ dieselbe Bedeutung haben wie in Formel (I),
mit Orthoformiaten der Formel **(III)** 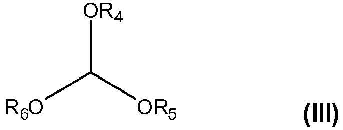 mit
- R₄, R₅, R₆ =: unabhängig voneinander (C₁-C₁₅)-Alkyl, (C₃-C₁₅)-Cycloalkyl oder (C₆-C₁₄)-Aryl, wobei diese substituiert oder unsubstituiert sind,
und Alkoholen und/oder Thioalkoholen der Formel **(IV)** 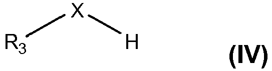 mit
- R₃ =: (C₁-C₁₅)-Alkyl, (C₃-C₁₅)-Cycloalkyl oder (C₆-C₁₄)-Aryl, wobei diese substituiert oder unsubstituiert sind, und
- X =: O, S,
in Gegenwart eines Katalysators, ausgewählt aus freien oder festphasengebundenen organischen Sulfonsäuren, Schwefelsäure, Hydrogensulfate, organischen oder anorganischen Phosphor-haltigen Säuren, deren Dihydrogen- und Hydrogensalze, sowie rauchender Salpetersäure und/oder Borsäure, zum entsprechenden Acetal umgesetzt wird und anschließend eine Eliminierung des Alkohols oder Thiols aus dem entsprechenden Acetal zum substituierten 1,4-Chinonmethid der Formel **(I)** erfolgt. Weiterer Gegenstand dieser Erfindung ist ein Verfahren zur Herstellung des 3,5-disubstituierten 4-Hydroxybenzaldehyds der Formel **(II),** das sich dadurch auszeichnet, dass 2,6-disubstituierte Phenole gemäß der Formel **(VII)** 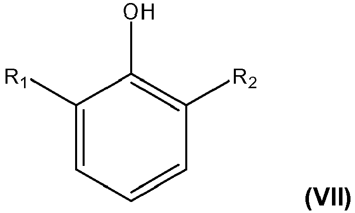 wobei R₁ und R₂ dieselbe Bedeutung haben wie in Formel **(I),** mit Urotropin in einem Lösemittelgemisch bestehend aus Eisessig und Wasser bei Temperaturen, die während der gesamten Reaktionszeit mindestens 2°C unterhalb der Rückflusstemperatur liegen, umgesetzt wird und das molare Verhältnis des 2,6-disubstituierten Phenols zum Urotropin von 1 : 1 bis 1 : 0,8 ist. Ebenfalls Gegenstand dieser Erfindung ist Verfahren zur Herstellung von substituierten 1,4-Chinonmethiden der Formel **(V)** 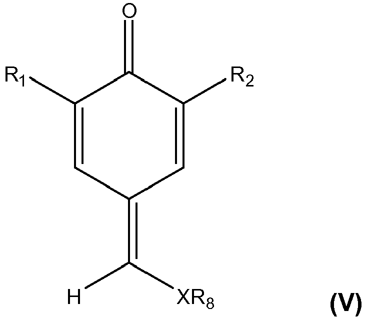 mit
- R₁, R₂ =: unabhängig voneinander Wasserstoff, (C₁-C₁₅)-Alkyl, (C₃-C₁₅)-Cycloalkyl oder (C₆-C₁₄)-Aryl, wobei diese substituiert oder unsubstituiert sind,
- R₈ =: (C₃-C₁₅)-Alkyl, (C₃-C₁₅)-Cycloalkyl oder (C₆-C₁₄)-Aryl, wobei diese substituiert oder unsubstituiert sind, und
- X =: O, S,
das sich dadurch kennzeichnet, dass ein substituiertes 1,4-Chinonmethid der Formel **(I)** mit R₇ = unsubstituierte (C₁-C₂)-Alkylgruppe und X = O, wobei R₁ und R₂ dieselbe Bedeutung haben wie in Formel **(V),** in Gegenwart eines Alkohols bzw. Thioalkohols der Formel **(VI)** 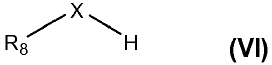 mit
- R₈ =: (C₃-C₁₅)-Alkyl, (C₃-C₁₅)-Cycloalkyl oder (C₆-C₁₄)-Aryl, wobei und diese substituiert oder unsubstituiert sind, und
- X =: O, S,
umgesetzt wird.

Für die Acetalbildung in dem erfindungsgemäßen Verfahren zur Herstellung von substituierten 1,4-Chinonmethiden der Formel **(I)** werden vorzugsweise 3,5-disubstituierten 4-Hydroxybenzaldehyde der Formel **(II)** als Edukt eingesetzt, die (C₁-C₁₅)-Alkyl-, (C₃-C₁₅)-Cycloalkyl- und/oder (C₆-C₁₄)-Arylgruppen als Substituenten R₁ und R₂ aufweisen, bevorzugt weisen die eingesetzten 3,5-disubstituierten 4-Hydroxybenzaldehyde (C₁-C₄)-Alkyl- und/oder (C₃-C₁₅)-Cycloalkylgruppen als Substituenten R₁ und R₂ auf. Besonders bevorzugt werden 3,5-disubstituierte 4-Hydroxybenzaldeyde mit (C₁-C₄)-Alkylgruppen, ganz besonders bevorzugt mit verzweigten (C₃-C₄)-Alkylgruppen, wie tert.-Butyl- oder *iso*-Propylgruppen, als Substituenten R₁ und R₂ eingesetzt.

Insbesondere weisen die eingesetzten 3,5-disubstituierten 4-Hydroxybenzaldehyde als Substituenten R₁ und R₂ unsubstituierte Gruppen auf.

Im Sinne der Erfindung werden unter Substituenten Gruppen, ausgewählt aus -COOR, - OH, -OR, -Halogen, -NR₂, =O und -CO-NR₂, wobei R = Wasserstoff, (C₁-C₁₅)-Alkyl-, (C₃-C₁₅)-Cycloalkyl- und/oder (C₆-C₁₄)-Arylgruppen ist, verstanden, die wiederum durch mindestens einen dieser Substituenten substituiert sein können.

Als weiteres Edukt werden für die Acetalbildung in dem erfindungsgemäßen Verfahren Orthoformiate der Formel **(III)** eingesetzt, wobei die Substituenten R₄, R₅ und R₆ vorzugsweise (C₁-C₁₅)-Alkyl- oder (C₃-C₁₅)-Cycloalkylgruppen und bevorzugt (C₁-C₄)-Alkylgruppen sind. Besonders bevorzugt sind diese Substituenten R₄, R₅ und R₆ unsubstituiert. In einer besonders bevorzugten Ausführungsform des erfindungsgemäßen Verfahrens werden Orthoformiate eingesetzt, die als Substituenten R₄, R₅ und R₆ (C₁-C₂)-Alkylgruppen aufweisen. Ganz besonders werden Orthoformiate eingesetzt, deren Substituenten R₄, R₅ und R₆ alle identisch sind. Insbesondere wird in dem erfindungsgemäßen Verfahren Trimethylorthoformiat eingesetzt.

Neben den 3,5-disubstituierten 4-Hydroxybenzaldehyden und den Orthoformiate werden für die Acetalbildung des erfindungsgemäßen Verfahrens auch Alkohole und/oder Thioalkohole der Formel **(IV)** eingesetzt, wobei der Substituent R₃ vorzugsweise unsubstituiert ist. Bevorzugt ist der Substituent R₃ eine (C₁-C₁₅)-Alkyl- oder (C₃-C₁₅)-Cycloalkylgruppe und besonders bevorzugt eine (C₁-C₄)-Alkylgruppe.

In einer besonders bevorzugten Ausführungsform des erfindungsgemäßen Verfahrens werden Alkohole eingesetzt, besonders bevorzugt werden Alkohole mit einer Phenyl- oder (C₁-C₁₅)-Alkylgruppe und ganz besonders bevorzugt mit einer (C₁-C₄)-Alkylgruppe als Substituent R₃ eingesetzt. Insbesondere wird Methanol oder Ethanol in dem erfindungsgemäßen Verfahren eingesetzt.

In dem erfindungsgemäßen Verfahren werden vorzugsweise Orthoformiate und Alkohole und/oder Thioalkohole eingesetzt, wobei die Substituenten R₄, R₅ und R₆ des Orthoformiats identisch sind mit dem Substituenten R₃ des Alkohols und/oder Thioalkohols.

Unterscheiden sich die Substituenten R₃ und R₄, R₅ und R₆, so können Mischungen aus verschiedenen substituierten 1,4-Chinonmethiden entstehen. Bei dem Einsatz eines Thioalkohols bildet sich überwiegend das Chinonmethid mit X = S und R₇ = R₃ des Thioalkohols. Hingegen bildet sich bei dem Einsatz von Orthoformiaten und (Thio-)alkoholen mit unterschiedlichen Substituenten R₃ und R₄, R₅ und R₆ vorzugsweise das entsprechend substituierte 1,4-Chinonmethid gemäß Formel **(I)** des am schwerflüchtigsten (Thio-)alkohols.

Als Katalysator werden in dem erfindungsgemäßen Verfahren vorzugsweise freie oder festphasengebundene organische Sulfonsäuren, Schwefelsäure, Hydrogensulfate, organische oder anorganische Phosphor-haltige Säuren, deren Dihydrogen- und Hydrogensalze, sowie rauchende Salpetersäure und/oder Borsäure eingesetzt, bevorzugt werden freie oder festphasengebundene organische Sulfonsäuren, Schwefelsäure und/oder Hydrogensulfate eingesetzt und besonders bevorzugt werden Alkylbenzolsulfonsäuren, Polymere, die Sulfonsäuregruppen aufweisen, oder Hydrogensulfate der Alkali- und Erdalkalimetalle eingesetzt. Ganz besonders bevorzugt werden als Katalysatoren Hydrogensulfate der Alkali- und Erdalkalimetalle eingesetzt, insbesondere Kaliumhydrogensulfat oder Natriumhydrogensulfat.

Als organische Sulfonsäure können Alkylbenzolsulfonsäuren, wie beispielsweise p-Toluolsulfonsäure oder Dodecylbenzolsulfonsäure, oder Polymere, die Sulfonsäuregruppen aufweisen, eingesetzt werden.

Der Einsatz von Feststoffen als Katalysator hat den Vorteil, dass dieser Katalysator einfach, beispielsweise mittels Filtration, vom Reaktionsgemisch abgetrennt werden kann. Im Vergleich zu den Verfahren mit Ammoniumchlorid - gemäß dem Stand der Technik - ermöglicht das erfindungsgemäße Verfahren eine großtechnische Produkt ohne Halogenide und somit bedarf es keines aufwändigen Korrosionsschutzes der Anlagenbauteile. Ferner handelt es sich bei den in dem erfindungsgemäßen Verfahren eingesetzten Katalysatoren um preiswerte, halogenfreie und nicht-toxische Säuren.

Das molare Verhältnis des 3,5-disubstituierten 4-Hydroxybenzaldehyds der Formel **(II)** zum Katalysator beträgt in dem erfindungsgemäßen Verfahren vorzugsweise von 1 : 0,0002 bis 1 : 0,5, bevorzugt von 1 : 0,0005 bis 1 : 0,2, besonders bevorzugt von 1 : 0,001 bis 1 : 0,1 und ganz besonders bevorzugt von 1 : 0,005 bis 1 : 0,05.

Das erfindungsgemäße Verfahren zeichnet sich dadurch aus, dass das molare Verhältnis des 3,5-disubstituierten 4-Hydroxybenzaldehyds zum Orthoformiat vorzugsweise von 1 : 0,5 bis 1 : 10 ist, bevorzugt ist das Verhältnis 1 : 0,9 bis 1 : 5 und besonders bevorzugt von 1 : 1 bis 1 : 2. Durch das Absenken des relativ teuren Orthoformiats können die Betriebskosten einer großtechnischen Anlage gesenkt werden, ohne dass die Umsätze dadurch geschmälert werden.

Die Acetalbildung kann in dem erfindungsgemäßen Verfahren sowohl mit einem zusätzlichen Lösemittel (A) als auch ohne ein zusätzliches Lösemittel durchgeführt werden. Als zusätzliches Lösemittel (A) eignen sich Lösemittel, die gegenüber den eingesetzten Edukten 4-Hydroxybenzaldehyd, Alkohol, Thiol und Orthoformiat sowie gegenüber dem (Thio-)Acetal inert sind, bevorzugt werden aromatische Lösemittel, wie Toluol, Ethylbenzol und/oder Xylole, eingesetzt.

In einer besonders bevorzugten Ausführungsform des erfindungsgemäßen Verfahrens erfolgt die Umsetzung des 4-Hydroxybenzaldehyds zum (Thio-)Acetal in Abwesenheit eines zusätzlichen Lösemittels (A). Auf diese Weise kann die Raum-Zeit-Ausbeute des erfindungsgemäßen Verfahrens verbessert werden.

Die Umsetzung des 4-Hydroxybenzaldehyds zum (Thio-)Acetal erfolgt in dem erfindungsgemäßen Verfahren vorzugsweise durch Erhitzen unter Rückfluss für einige Minuten bis Stunden, bevorzugt für 0,5 bis 10 Stunden und besonders bevorzugt für 1 bis 5 Stunden.

Die Umsetzung des 3,5-disubstituierten 4-Hydroxybenzaldehyds zum entsprechenden (Thio-)Acetal kann bei verschiedenen Drücken durchgeführt werden, vorzugsweise erfolgt diese Verfahrensstufe bei Atmosphärendruck. Bei dem Einsatz von einigen Thioalkoholen ist aufgrund deren niedriger Siedepunkte das Arbeiten unter Druck empfehlenswert.

Das entstehende (Thio-)Acetal kann in dem erfindungsgemäßen Verfahren durch die üblichen Isolationsschritte, wie Einengen der Lösung, Extraktion, Filtration, Kristallisation, etc., isoliert werden. Vorzugsweise wird das (Thio-)Acetal vor der Eliminierung des Alkohols nicht isoliert, sondern direkt aus der Lösung zum gewünschten Produkt - dem substituierten 1,4-Chinonmethid gemäß Formel **(I)** - umgesetzt.

In dem erfindungsgemäßen Verfahren sollte vor der Eliminierung des (Thio-)Alkohols aus dem (Thio-)Acetal das überschüssige Orthoformiat und der überschüssigen Alkohol bzw. Thioalkohol - vorzugsweise destillativ - entfernen werden. Falls das (Thio-)Acetal nicht isoliert werden soll, ist es empfehlenswert vor dieser destillativen Abtrennung des Orthoformiats und des Alkohols bzw. Thioalkohols ein zusätzliches Lösemittel (B) zuzugeben. Damit kann erreicht werden, dass das (Thio-)Acetal auch nach der destillativen Abtrennung des Orthoformiats und des Alkohols bzw. Thioalkohols in Lösung bleibt.

Als zusätzliches Lösemittel (B) eignen sich Lösemittel, die einen höheren Siedepunkt als den des eingesetzten Alkohols und/oder Thioalkohols aufweisen und inert gegenüber dem (Thio-)Acetal und dem zu bildenden substituierten 1,4-Chinonmethid sind. Der Siedepunkt des eingesetzten Lösemittel (B) sollte mindestens 100°C, bevorzugt von 110°C bis 250°C betragen. Ferner sollte dieses Lösemittel (B) in der Lage sein, das gebildete Acetal in Lösung zu halten. Insbesondere werden in dem erfindungsgemäßen Verfahren aromatische Lösemittel, wie beispielsweise Toluol, Ethylbenzol, o-, m- oder p-Xylol, und Mischung dieser und anderer aromatischen Lösemittel eingesetzt. Es können auch Mischungen von aromatischen Kohlenwasserstoffen mit einem entsprechenden definierten Siedepunktsbereich als zusätzliches Lösemittel (B) eingesetzt werden.

Diese Abtrennung mittels Destillation des überschüssigen Orthoformiats und des überschüssigen Alkohols bzw. Thioalkohols wird vorzugsweise solange durchgeführt, bis das Reaktionsgemisch den Siedepunkt des zusätzlichen Lösemittels (B) erreicht hat. Diese Abtrennung kann sowohl unter Atmosphärendruck als auch unter vermindertem Druck erfolgen.

In dem erfindungsgemäßen Verfahren sollte der Katalysator ebenfalls vor der thermischen Eliminierung des (Thio-)Alkohols aus dem (Thio-)Acetal abgetrennt werden, beispielsweise mittels mechanischer Trennverfahren. Dieses Verfahren eignet sich vor allem bei Katalysatoren, die als Feststoff vorliegen. Geeignete mechanische Trennverfahren sind hierfür Filtration, Sedimentation oder Zentrifugation mit anschließendem Dekantieren. Insbesondere bei Katalysatoren, die als Feststoff vorliegen, empfiehlt es sich, diesen vor der thermischen Eliminierung des (Thio-)Alkohols aus dem (Thio-)Acetal zu entfernen, damit die Kolonne nicht durch Feststoffeinträge verunreinigt wird.

Bei dem Einsatz von einem flüssigen Katalysator oder für nicht abgetrennte Spuren fester Katalysatoren werden diese vorzugsweise mit einer Base neutralisiert. Besonders bevorzugt werden hierzu nicht-nukleophile oder sterisch gehinderte Amine sowie anorganische Salze, wie zum Beispiel Carbonate, eingesetzt. Eine Abtrennung des Neutralisationsprodukts ist nicht unbedingt notwendig.

Die Reihenfolge der Abtrennung oder Neutralisation des Katalysators, der Zugabe eines Lösemittels (B) und der Entfernung des überschüssigen (Thio-)Alkohols und des überschüssigen Orthoformiats kann abhängig vom eingesetzten Katalysator, (Thio-)Alkohol und Orthoformiats ohne Umsatzeinbußen je nach Bedarf beliebig variiert werden.

Um die Eliminierung des (Thio-)Alkohols aus dem (Thio-)Acetal durchzuführen, wird das acetalhaltige Reaktionsgemisch des erfindungsgemäßen Verfahren vorzugsweise auf die Siedetemperatur des Lösemittels (B) erhitzt, bevorzugt auf mindestens 100°C, besonders bevorzugt auf 110 bis 250°C, wobei der freiwerdende (Thio-)Alkohol vorzugsweise unmittelbar nach seinem Entstehen mittels chemischer und/oder physikalischen Methoden aus dem Reaktionsgemisch entfernt wird. Der durch thermische Induktion eliminierte (Thio-)Alkohol kann durch übliche Methoden aus dem Reaktionsgemisch entfernt werden. So kann der freiwerdende Alkohol durch Zugabe von geeigneten Reagenzien, wie beispielsweise Anhydriden, chemisch gebunden werden. Aber auch physikalische Methoden, wie beispielsweise der Einsatz eines Molekularsiebes bei kurzkettigen (Thio-)Alkoholen, sind denkbar.

Im erfindungsgemäßen Verfahren erfolgt die Abtrennung des freiwerdenden (Thio-)Alkohols vorzugsweise mittels Destillation. Hierbei wird das acetalhaltige Reaktionsgemisch auf mindestens 100°C, bevorzugt auf 110 bis 250°C, erhitzt, wobei ein zusätzliches Lösemittel (C) kontinuierlich zudosiert wird, während gleichzeitig der freiwerdende Alkohol und/oder Thioalkohol zusammen mit dem zusätzlichen Lösemittel (C) aus dem Reaktionsgemisch entfernt wird. Besonders bevorzugt wird hierbei soviel zusätzliches Lösemittel (C) dem Reaktionsgemisch zudosiert, wie an Destillat, weitgehend bestehend aus (Thio-)Alkohol und Lösemittel (A, B und C) abdestilliert wird. Das zusätzliche Lösemittel (C) dient hierbei dazu, den überschüssigen Alkohol und/oder Thioalkohol aus dem Reaktionsgemisch leichter entfernen zu können und somit die Eliminierung des (Thio-)Alkohols aus dem (Thio-)Acetal zu erzielen. Es ist auch denkbar, die Eliminierung des Alkohols bei tieferen Temperaturen und gleichzeitiger Druckreduktion durchzuführen. Dieser Verfahrensschritt hat neben der Gleichgewichtsverschiebung zu Gunsten des substituierten 1,4-Chinonmethids gemäß Formel **(I)** auch den Vorteil, dass Spuren von Wasser ebenfalls aus dem Reaktionsgemisches entfernt werden und somit die Rückreaktion des substituierten 1,4-Chinonmethids zum 4-Hydroxybenzaldehyd weitgehend unterbunden werden kann.

Als zusätzliches Lösemittel (C) eignen sich auch hier Lösemittel, die einen Siedepunkt von mindestens 100°C, bevorzugt von 110°C bis 250°C aufweisen und inert gegenüber dem (Thio-)Acetal und dem substituierten 1,4-Chinonmethid gemäß Formel **(I)** sind. Insbesondere werden in dem erfindungsgemäßen Verfahren aromatische Lösemittel, wie beispielsweise Toluol, Ethylbenzol, o-, m- oder p-Xylol, und Mischung dieser aromatischen Lösemittel eingesetzt. Es können auch Mischungen von aromatischen Kohlenwasserstoffen mit einem entsprechenden definierten Siedepunktsbereich als zusätzliches Lösemittel (C) eingesetzt werden. In einer ganz besonders bevorzugten Ausführungsform des erfindungsgemäßen Verfahrens sind die Lösemittel (A), (B) und (C) identisch.

In einer weiteren Ausführungsform des erfindungsgemäßen Verfahrens werden zwei verschiedene Lösemittel eingesetzt, wobei dann das Lösemittel (C) vorzugsweise einen höheren Siedepunkt als das Lösemittel (B) aufweist. Dies ist dann von Vorteil, wenn das substituierte 1,4-Chinonmethid für seine weitere Verwendung in einem Lösemittel vorliegen soll, das für die Destillation des überschüssigen Orthoformiats und des überschüssigen Alkohols bzw. Thioalkohols weniger geeignet ist.

Für die Herstellung von substituierten 1,4-Chinonmethiden der Formel **(V)** 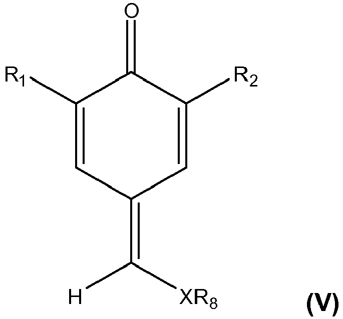 mit
- R₁, R₂ =: unabhängig voneinander Wasserstoff, (C₁-C,₅)-Alkyl, (C₃-C₁₅)-Cycloalkyl oder (C₆-C₁₄)-Aryl, wobei diese substituiert oder unsubstituiert sind,
- R₈ =: (C₃-C₁₅)-Alkyl, (C₃-C₁₅)-Cycloalkyl oder (C₆-C₁₄)-Aryl, wobei diese substituiert oder unsubstituiert sind, und
- X =: O, S,
kann das - vorzugsweise durch das erfindungsgemäße Verfahren entstandene - substituierte 1,4-Chinonmethid der Formel **(I)** mit R₇ = unsubstituierte (C₁-C₂)-Alkylgruppe und X = O, wobei R₁ und R₂ dieselbe Bedeutung wie in Formel **(V)** haben, in Gegenwart eines Alkohols bzw. Thioalkohols der Formel **(VI)** 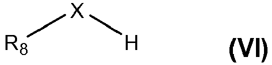 mit
- R₈ =: (C₃-C₁₅)-Alkyl, (C₃-C₁₅)-Cycloalkyl oder (C₆-C₁₄)-Aryl, wobei diese substituiert oder unsubstituiert sind,
- X =: O, S,
umgesetzt werden.

Hierbei wird das Reaktionsgemisch bestehend aus dem substituierten 1,4-Chinonmethid der Formel **(I)** mit R₇ = unsubstituierte (C₁-C₂)-Alkylgruppe und X = O, wobei R₁ und R₂ dieselbe Bedeutung wie in Formel **(V)** haben, und dem (Thio-)Alkohol der Formel **(VI)** des erfindungsgemäßen Verfahren vorzugsweise auf Siedetemperatur des entsprechenden Lösemittels erhitzt, bevorzugt auf mindestens 100°C, besonders bevorzugt auf 110 bis 250°C, wobei der Methanol und/oder Ethanol unmittelbar mittels chemischer und/oder physikalischen Methoden aus dem Reaktionsgemisch entfernt wird. Der durch thermische Induktion eliminierte Methanol und/oder Ethanol kann durch übliche Methoden aus dem Reaktionsgemisch entfernt werden. So kann der freiwerdende Methanol und/oder Ethanol durch Zugabe von geeigneten Reagenzien, wie beispielsweise Anhydriden, chemisch gebunden werden. Aber auch physikalische Methoden, wie beispielsweise der Einsatz eines Molekularsiebes, sind denkbar.

Im erfindungsgemäßen Verfahren erfolgt diese Abtrennung des freiwerdenden Methanol und/oder Ethanol vorzugsweise mittels Destillation. Hierbei wird das Reaktionsgemisch auf mindestens 100°C bevorzugt auf 110 bis 250°C, erhitzt, wobei ein zusätzliches Lösemittel (D) kontinuierlich zudosiert, während gleichzeitig der freiwerdende Methanol und/oder Ethanol zusammen mit dem zusätzlichen Lösemittel (D) aus dem Reaktionsgemisch entfernt wird. Besonders bevorzugt wird hierbei soviel zusätzliches Lösemittel (D) dem Reaktionsgemisch zudosiert, wie an Methanol und/oder Ethanol und Lösemitteln abdestilliert wird.

Als zusätzliches Lösemittel (D) eignen sich auch hier Lösemittel, die einen Siedepunkt von mindestens 100°C, bevorzugt von 110°C bis 250°C aufweisen und inert gegenüber Reaktionsbeteiligten als auch dem gewünschten Produkt sind. Insbesondere werden in dem erfindungsgemäßen Verfahren aromatische Lösemittel, wie beispielsweise Toluol, Ethylbenzol, o-, m- oder p-Xylol, und Mischung dieser aromatischen Lösemittel eingesetzt. Es können auch Mischungen von aromatischen Kohlenwasserstoffen mit einem entsprechenden definierten Siedepunktsbereich als zusätzliches Lösemittel (D) eingesetzt werden. In einer ganz besonders bevorzugten Ausführungsform des erfindungsgemäßen Verfahrens sind die eingesetzten Lösemittel (A), (B), (C) und (D) identisch.

Auf diese Weise sind substituierte 1,4-Chinonmethide gemäß der Formel **(V)** mit einem Substituenten R₈, der mehr als zwei Kohlenstoffatome aufweist, leicht zugänglich. Diese Variante des erfindungsgemäßen Verfahrens hat zum Vorteil, dass aus einem einzigen Zwischenprodukt dem substituierten 1,4-Chinonmethid der Formel **(I)** mit R₇ = unsubstituierte (C₁-C₂)-Alkylgruppe und X = O eine Vielzahl an Derivaten hergestellt werden können, wobei sich die einzelnen Herstellverfahren nur in dem letzten Verfahrensschritt unterscheiden. Dies ist insbesondere in der pharmazeutischen Wirkstoff-Forschung von besonderem Interesse.

Je nach weiterer Anwendung kann die mittels der erfindungsgemäßen Verfahren erhaltene Chinonmethid-Lösung direkt weiter verwendet werden. Für den Fall, dass die Chinonmethid-Lösung nicht direkt eingesetzt werden kann, kann das substituierte 1,4-Chinonmethid durch Abkühlen der Produktmischung, Kristallisation und Abtrennen des Produkts isoliert und ggf. aufgereinigt werden, beispielsweise durch Umkristallisation. Es kann auch hilfreich sein, das Lösemittel für den Kristallisationsprozess komplett zu entfernen, und gegen ein Lösemittel auszutauschen, in dem das substituierte 1,4-Chinonmethid eine geringere Löslichkeit aufweist. Auf diese Weise kann der Kristallisationsprozess vereinfacht oder beschleunigt werden. Das substituierte 1,4-Chinonmethid kann auch durch ein vollständiges oder teilweises Entfernen des Lösemittels gewonnen werden.

Das in dem erfindungsgemäßen Verfahren eingesetzte 3,5-disubstituierten 4-Hydroxybenzaldehyd der Formel **(II)** kann sowohl ausgehend vom 2,6-disubstituierten Phenol als auch ausgehend vom 2,6-disubstituierten 4-Methylphenol gemäß zahlreichen Verfahren gemäß dem Stand der Technik hergestellt werden. Als besonders geeignet hat sich ein Verfahren herausgestellt, das sich dadurch auszeichnet, dass 2,6-disubstituiertes Phenol gemäß der Formel **(VII)** mit Urotropin in einem Lösemittelgemisch bestehend aus Eisessig und Wasser bei Temperaturen, die während der gesamten Reaktionszeit mindestens 2°C unterhalb der Rückflusstemperatur liegen, umsetzt wird.

Als Edukte werden für das erfindungsgemäße Verfahren zur Herstellung des 3,5-disubstituierten 4-Hydroxybenzaldehyds vorzugsweise 2,6-disubstituiertes Phenol oder Mischungen solcher Verbindungen eingesetzt, wobei die an Position 2 und 6 befindlichen Substituenten den Substituenten R₁ und R₂ in der Formel **(II)** entsprechen.

Bevorzugt werden 2,6-disubstituierte Phenole eingesetzt, die (C₁-C₁₅)-Alkyl-, (C₃-C₁₅)-Cycloalkyl- und/oder (C₆-C₁₄)-Arylgruppen, besonders bevorzugt (C₁-C₄)-Alkyl- und/oder (C₃-C₁₅)-Cycloalkylgruppen als Substituenten R₁ und R₂ aufweisen. Ganz besonders bevorzugt werden 2,6-disubstituierte Phenole mit (C₁-C₄)-Alkylgruppen als Substituenten R₁ und R₂ eingesetzt. In einer besonders bevorzugten Ausführungsform des erfindungsgemäßen Verfahrens werden 2,6-disubstituierte Phenole eingesetzt, die verzweigte (C₃-C₄)-Alkylgruppen, wie *tert*.-Butyl- oder *iso*-Propylgruppen, aufweisen.

Insbesondere weisen die eingesetzten 2,6-disubstituierten Phenole als Substituenten R₁ und R₂ unsubstituierte Gruppen auf.

Dieses erfindungsgemäße Verfahren zeichnet sich ebenfalls durch seine Halogenfreiheit aus, da vorzugsweise keine halogenhaltigen Verbindungen eingesetzt werden.

Das erfindungsgemäße Verfahren zeichnet sich dadurch aus, dass das molare Verhältnis des 2,6-disubstituierten Phenols zum Urotropin vorzugsweise kleiner gleich 1 : 1 ist, bevorzugt ist ein Verfahren bei dem dieses molare Verhältnis von 1 :1 bis 1 : 0,8 ist.

Das Mengenverhältnis von Eisessig zu Wasser wird in diesem erfindungsgemäßen Verfahren vorzugsweise so gewählt, dass eine Reaktionstemperatur von 115°C +/- 10 °C eingestellt werden kann, ohne dass Wasser abdestilliert werden muss.

Dadurch kann ein häufig im Stand der Technik genannter Verfahrensschritt entfallen, wodurch das erfindungsgemäße Verfahren effektiver gestaltet werden kann. Bevorzugt wird in dem erfindungsgemäßen Verfahren ein Molverhältnis von Essigsäure zu Wasser von 1:1 bis 20:1, besonders bevorzugt von 1,1 :1 bis 10 : 1 und ganz besonders bevorzugt von 1,2 : 1 bis 5 : 1 eingestellt.

Die Zusammengabe des 2,6-disubstituierten Phenols, des Eisessigs, des Wassers und des Urotropins kann in jeder beliebigen Reihenfolge erfolgen. Vorzugsweise wird das 2,6-disubstituierte Phenol in Eisessig gelöst, Urotropin und schließlich das Wasser zugegeben. Auch kann die Mischung der Komponenten entweder bei Raumtemperatur oder bei erhöhten Temperaturen erfolgen.

Die Umsetzung des 2,6-disubstituierten Phenols zum 3,5-disubstituierten 4-Hydroxybenzaldehyd erfolgt in diesem erfindungsgemäßen Verfahren während der gesamten Reaktionszeit vorzugsweise bei einer Temperatur wenige Grade unterhalb der Rückflusstemperatur, vorzugsweise liegt die Reaktionstemperatur mindestens 2°C, bevorzugt mindestens 3°C und besonders bevorzugt 5°C unterhalb der Rückflusstemperatur. Dies hat den Vorteil, dass einerseits die Reaktionstemperatur für eine nahezu vollständige Umsetzung des 2,6-disubstituierten Phenols zum 3,5-disubstituierten 4-Hydroxybenzaldehyd ausreichend ist, andererseits die Temperatur nicht ausreichend ist, das Reaktionsgemisch zu refluxieren und so mit dem sich vorzugsweise an der Flüssigkeitsoberfläche ansammelnde Produkt, das oft als Feststoff vorliegt, die Kolonne zu verschmutzen oder gar zu blockieren. Insbesondere bei großtechnischen Verfahren ist dies ein unerwünschter Effekt. Ferner hat sich gezeigt, dass durch ein Verfahren, das mit einer Reaktionstemperatur unterhalb der Rückflusstemperatur arbeitet, auch die Reinheit des substituierten 1,4-Chinonmethids verbessern lässt. In einer besonders bevorzugt Ausführungsform dieses erfindungsgemäßen Verfahrens wird das Reaktionsgemisch während der gesamten Reaktionszeit auf eine Temperatur von 115°C +/- 10°C erhitzt. Die Reaktionstemperatur liegt während der gesamten Reaktionszeit vorzugsweise nicht niedriger als 10°C unterhalb der Rückflusstemperatur.

Im Rahmen dieser Erfindung wird unter der gesamten Reaktionszeit die Zeitspanne verstanden, in der die gewünschte Reaktionstemperatur für die Umsetzung des 2,6-disubstituierten Phenols mit dem Urotropin erreicht und gehalten wird. Die Aufheiz- und Abkühlphase gehören im Rahmen dieser Erfindung nicht zur gesamten Reaktionszeit, auch wenn in diesen Phasen schon oder noch Umsetzungen des 2,6-disubstituierten Phenols beobachtet werden können.

Vorzugsweise wird das Reaktionsgemisch bei der Umsetzung des 2,6-disubstuierten Phenols und des Urotropins in diesem erfindungsgemäßen Verfahren für 1 bis 10 Stunden, bevorzugt für 2 bis 7 Stunden und besonders bevorzugt für 3 bis 6 Stunden auf die gewünschte Reaktionstemperatur erhitzt.

Aufgrund der unterschiedlichen Substituenten in Position R₁ und R₂ kann die Löslichkeit bzw. der Schmelzpunkt der 3,5-disubstituierten 4-Hydroxybenzaldehyde sehr unterschiedlich sein. Abhängig vom Substitutionsmuster des 4-Hydroxybenzaldehydes können verschiedene Methoden zur Isolation des 3,5-disubstituierten 4-Hydroxybenzaldehyds in diesem erfindungsgemäßen Verfahren eingesetzt werden.
(A) Abfiltrieren des ausgefallenen Feststoffs, wobei nach dem Filtrieren das erhaltene Filtrat einer weiteren Umsetzung von 2,6-disubstituierten Phenolen zum 3,5-disubstituierten 4-Hydroxybenzaldehyd zugeführt werden kann.
(B) Zugabe von Wasser, um auf diese Weise das 3,5-disubstituierte 4-Hydroxybenzaldehyd auszufällen, wobei die weitere Aufarbeitung gemäß (A) erfolgt.
(C) Extraktion mit einem geeigneten Lösemittel, anschließend das Extrakt mit Wasser gewaschen und das Lösemittel destillativ entfernt wird. Als Lösemittel können hierbei Lösemittel eingesetzt werden, die nicht oder nicht gut mit Wasser mischbar sind, bevorzugt werden hierfür aromatische Lösemittel, wie Toluol, Ethylbenzol, Xylole, oder Mischungen dieser genannten Lösemittel eingesetzt.
(D) Abdestillation von Essigsäure und Wasser, wobei anschließend der Rückstand überwiegend bestehend aus dem 3,5-disubstituierten 4-Hydroxybenzaldehyd mit Wasser gewaschen werden kann, um die bei der Reaktion entstandenen Salze ebenfalls zu entfernen.

Die nachfolgenden Beispiele sollen die erfindungsgemäßen Verfahren näher erläutern, ohne dass die Erfindung auf diese Ausführungsform beschränkt sein soll.

### Beispiele 1-4:

7 g (30 mmol) an 3,5-Di-*tert*.-butyl-4-hydroxybenzaldehyd werden in einem Reaktionskolben vorgelegt und mit einem Gemisch bestehend aus 14,5 ml Methanol und 14,2 g Trimethylorthoformiat versetzt. Anschließend werden 5 mmol des Katalysators gemäß der Tabelle 1 zugegeben. Das Reaktionsgemisch wird unter Rühren auf Rückfluss erhitzt. Nach drei Stunden wird das Reaktionsgemisch auf Raumtemperatur abgekühlt und der Umsatz des Aldehyds mittels Gaschromatographie bestimmt. Die Umsätze des Aldehyds in Abhängigkeit von dem eingesetzten Katalysator zeigt die Tabelle 1.

**Tabelle 1:**

| **Beispiel** | **Katalysator** | **Umsatz des Aldehyds** (in GC-%) |
|---|---|---|
| VB 1 | Ammoniumchlorid | 98,6 |
| 1 | p-Toluolsulfonsäure | 95,0 |
| 2 | Schwefelsäure | 91,1 |
| 3 | Kaliumhydrogensulfat | 99,4 |
| 4 | Sulfoniertes Polystyrol (Lewatit® K2649) | 98,6 |

| | | |
|---|---|---|
| VB: Vergleichsbeispiel | | |

Die Beispiele 1 bis 4 zeigen, dass besonders organische Sulfonsäuren als auch Schwefelsäure sowie deren Hydrogensalze geeignete Katalysatoren für das erfindungsgemäße Verfahren sind. Die Umsätze des Aldehyds liegen in allen Beispielen über 90%. Bei Beispiel 4 liegt der Umsatz in derselben Größenordnung wie bei einem Katalysator gemäß dem Stand der Technik (VB1), bei Beispiel 3 liegt der Umsatz sogar höher.

### Beispiele 3 - 6:

Die Versuchsdurchführung ist dieselbe, wie bereits bei den Beispielen 1-4 beschrieben, allerdings wird nun der Katalysator als auch die Menge des Katalysators entsprechend der Tabelle 2 variiert.

**Tabelle 2:**

| **Beispiel** | **Katalysator** | **Menge an Katalysator** (in mmol) | **molares Verhältnis Aldehyd zu Katalysator** | **Umsatz des Aldehyds** (in GC%) |
|---|---|---|---|---|
| VB 1 | NH₄Cl | 5 mmol | 1 : 0,17 | 98,6 |
| VB 2 | NH₄Cl | 2,5 mmol | 1 : 0,08 | 97,4 |
| 3 | KHSO₄ | 5 mmol | 1 : 0,17 | 99,4 |
| 5 | KHSO₄ | 1,1 mmol | 1 : 0,04 | 99,6 |
| 6 | KHSO₄ | 0,4 mmol | 1 : 0,01 | 99,9 |

| | | | | |
|---|---|---|---|---|
| VB: Vergleichsbeispiel | | | | |

Die Beispiele 3, 5 und 6 zeigen deutlich, dass durch eine Verringerung der molaren Menge an Katalysator in dem erfindungsgemäßen Verfahrens die Umsätze weiter gesteigert werden können. Im Gegensatz dazu zeigen die Vergleichsbeispiele 1 und 2, dass bei dem Einsatz von Ammoniumchlorid - dem Katalysator gemäß dem Stand der Technik - eine Verringerung der molaren Menge an Katalysator zu geringeren Umsätzen führt.

### Beispiele 7 - 11:

Die Versuchsdurchführung ist dieselbe, wie in Beispiel 5, allerdings wird nun die Menge an Methanol und Trimethylorthoformiat entsprechend der Tabelle 3 variiert.

**Tabelle 3:**

| **Beispiel** | **Methanol** | | **Trimethylorthoformiat** | | **Umsatz des Aldehyds** |
|---|---|---|---|---|---|
| | (in ml) | molares Verhältnis zum Aldehyd | (in g) | molares Verhältnis zum Aldehyd | (in GC%) |
| 7 | 13,3 | 10,96 | 14,1 | 4,42 | 99,6% |
| 8 | 17,8 | 14,62 | 10,5 | 3,31 | 99,6% |
| 9 | 22,2 | 18,27 | 7,0 | 2,21 | 99,6% |
| 10 | 26,7 | 21,93 | 3,5 | 1,10 | 99,5% |
| 11 | 28,9 | 23,75 | 1,8 | 0,55 | Kein vollständiger Umsatz Aldehyd löst sich nicht vollständig |

| | | | | | |
|---|---|---|---|---|---|
| VB: Vergleichsbeispiel | | | | | |

Die Beispiele 7 bis 11 zeigen, dass der Gehalt an Orthoformiat bis auf ein molares Verhältnis von Aldehyd zum Orthoformiat von 1: 1,1 herabgesenkt werden kann, ohne dass der Umsatz des Aldehyds beeinträchtigt wird.

### Beispiele 12 - Herstellung des Methoxy-substituierten Chinonmethids:

In einem 35 I-Glaskessel mit Rührwerk und Kühler werden 4,0 kg 3,5-Di-*tert*.-butyl-4-hydroxybenzaldehyd gegeben, 2 kg Trimethylorthoformiat und 6 kg Methanol zugesetzt und gemischt. Anschließend werden 45 g Natriumhydrogensulfat zugesetzt. Nun wird das Reaktionsgemisch für ca. 1 bis 2 Stunden unter Rückfluss erhitzt. Nach einer Stunde wird der Umsatz des Aldehyds mittels Gaschromatographie überprüft. Der eingesetzte Aldehyd hat sich bereits nach einer Stunde vollständig umgesetzt. Das Reaktionsgemisch wird abgekühlt und über einen Schenkfilter filtriert. Der Filterrückstand wird mit 8 kg Ethylbenzol nachgewaschen. Das Filtrat wird wieder in den Glasrührkessel zurück gegeben und das Gemisch aus Methanol, Trimethylorthoformiat und Ethylbenzol wird möglichst schnell abdestilliert. Anschließend wird mit der azeotropen Destillation begonnen, wobei kontinuierlich 300 bis 500 ml an Ethylbenzol pro Stunde zugesetzt werden und ebenso viel Destillat abgenommen werden. Nach 5 Stunden wird ein Umsatz von 70 % erreicht, nach 9 Stunden steigt der Umsatz zum gewünschten Chinonmethid auf über 90 %.

Das Reaktionsgemisch wird abgekühlt. Das gewünschte Chinonmethid fällt beim Abkühlen aus und kann mit einer Reinheit von > 98% isoliert werden.

### Beispiele 13 - Herstellung des Butoxy-substituierten Chinonmethids aus 3,5-Di-tert.-butyl-4-hydroxybenzaldehyd

7 g des 3,5-Di-*tert*.-butyl-4-hydroxybenzaldehyds werden mit 3,5 g Trimethylorthoformiat und 21 g n-Butanol versetzt. Anschließend werden 0,1 g Kaliumhydrogensulfat zugesetzt und das Reaktionsgemisch für 2 Stunden unter Rückfluss erhitzt. Der eingesetzte Aldehyd hat sich quantitativ umgesetzt.

Nun wird das Kaliumhydrogensulfat abfiltriert und der Filterrückstand mit 50 g Ethylbenzol nachgespült. Anschließend wird das Gemisch aus Methanol, Trimethylorthoformiat und Ethylbenzol abdestilliert bis eine Siedetemperatur von 130 °C erreicht wird. Dann wird ständig 100 ml Ethylbenzol pro Stunde zugesetzt und gleichzeitig die gleiche Menge an Destillat abgenommen.

Nach 6 Stunden hat sich das Butoxy-substituierte Chinonmethid zu 82% gebildet. Als Nebenprodukt sind 7 % des 3,5-Di-*tert*.-butyl-4-hydroxybenzaldehyd entstanden.

### Beispiele 14 - Herstellung des Butoxy-substituierten Chinonmethids aus dem Methoxy-substituierten Chinonmethid:

1 g des Methoxy-substituierten Chinonmethids (aus Beispiel 12) wird in 20 g Ethylbenzol gelöst. Dann wird 1 g n-Butanol zugesetzt. Das Reaktionsgemisch wird für zwei Stunden unter Rückfluss erhitzt. Laut der Analyse mittels Gaschromatographie haben sich 73 % des Methoxy-substituierten Chinonmethids zum Butoxy-substituierten Chinonmethid umgesetzt.

### Beispiele 15 - Herstellung eines Aroxy-substituierten Chinonmethids aus dem Methoxy-substituierten Chinonmethid:

1 g des Methoxy-substituierten Chinonmethids wird in 35 g Ethylbenzol gelöst. Anschließend werden 0,65 g 4-tert-Butylcatechol zugesetzt. Das Reaktionsgemisch wird für zwei Stunden unter Rückfluss erhitzt. Laut Analyse mittels Gaschromatographie haben sich 77 % des Methoxy-substituierten Chinonmethids zum mit 4-tert-Butylcatechol-substituierten Chinonmethid (Aryloxy-substituiert) umgesetzt.

### Beispiel 16 - 20 - Herstellung des 3,5-Di-tert.-butyl-4-hydroxybenzaldehyds

2,6-Di-*tert*.-butylphenol wird in Eisessig gelöst. Anschließend wird Urotropin und Wasser zugesetzt und das Reaktionsgemisch für 4 bis 5 Stunden bei maximal 2°C unterhalb der Rückflusstemperatur erhitzt. Das ausgefallene Produkt wird abfiltriert und mit Wasser und Methanol gewaschen und am Rotationsverdampfer getrocknet. Die molaren Verhältnisse der eingesetzten Edukte als auch die ermittelten Ausbeuten an 3,5-Di-*tert*.-butyl-4-hydroxybenzaldehyds bezogen auf 2,6-Di-tert-Butylphenol zeigt die Tabelle 4.

**Tabelle 4:**

| **Beispiel** | **Molares Verhältnis von** ... **zu 2,6-Di-*tert*.-butylphenol** | | **Molverhältnis Essigsäure zu Wasser** | **Ausbeute** (in % bezogen auf 2,6-Di-*tert*.-butylphenol) |
|---|---|---|---|---|
| | **Urotropin** | **Essigsäure** | | |
| 16 | 0,50 | 14,52 | 1,54 | 60,50 |
| 17 | 0,67 | 14,52 | 1,54 | 82,57 |
| 18 | 0,80 | 14,52 | 1,54 | 89,22 |
| 19 | 0,80 | 12,66 | 1,34 | 89,47 |
| 20 | 1 | 14,52 | 1,54 | 87,46 |

Die Beispiele 16 bis 19 zeigen unerwartet hohe Ausbeuten, die aufgrund der geringen Mengen an Urotropin nicht zu erwarten gewesen sind. Diese Beispiele zeigen, dass auch ein Verfahren mit einem molaren Verhältnis von Urotropin zum Phenol von kleiner 1 zu erstaunlich hohen Ausbeuten und Umsätzen führt.

### Beispiel 21 - 25 - Herstellung des 3,5-Di-tert.-butyl-4-hydroxybenzaldehyds (Variation der Reaktionstemperatur)

2,6-Di-*tert*.-butylphenol wird in Eisessig gelöst. Anschließend wird Urotropin und Wasser zugesetzt und das Reaktionsgemisch für 5,5 Stunden bei unterschiedlichen Temperaturen erhitzt. Das ausgefallene Produkt wird abfiltriert und mit Wasser und Methanol gewaschen und am Rotationsverdampfer getrocknet. Die jeweilige Reaktionstemperatur, Ausbeute und Reinheit zeigt die Tabelle 5.

**Tabelle 5:**

| **Beispiel** | **Molares Verhältnis von** ... **zu 2,6-Di-*tert*.-butylphenol** | | **Molverhältnis Essigsäure zu Wasser** | **Reaktions-temperatur** (in °C) | **Ausbeute** (in % bezogen auf 2,6-Di-*tert*.-butylphenol) | **Reinheit** (in %) |
|---|---|---|---|---|---|---|
| | **Urotropin** | **Essigsäure** | | | | |
| 21 | 1,00 | 7,26 | 1,54 | 118-140 | 91,4 | 72,0 |
| 22 | 1,00 | 7,26 | 1,54 | 118-126 | 92,6 | 96,5 |
| 23 | 1,00 | 7,26 | 1,54 | 117-120 | 90,1 | 99,9 |
| 24 | 1,00 | 14,52 | 1,54 | 118-124 | 88,7 | 98,6 |
| 25 | 1,00 | 14,52 | 1,54 | 109-118 | 83,1 | 45,1 |

Die Beispiele 21 - 25 zeigen, dass zu niedrige Reaktionstemperaturen zu Umsatzeinbußen, zu hohe Reaktionstemperaturen dagegen zu Reinheitsproblemen führen. Zusätzlich besteht bei zu hohen Reaktionstemperaturen ab der Rückflusstemperatur die Gefahr, dass Feststoffe die Kolonne verstopfen.

## Patentansprüche

1. Verfahren zur Herstellung von substituierten 1,4-Chinonmethiden der Formel 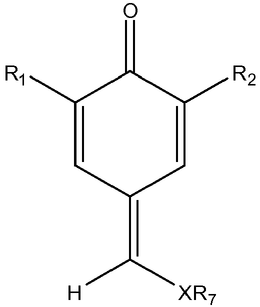 mit
R₁, R₂ = unabhängig voneinander Wasserstoff, (C₁-C₁₅)-Alkyl, (C₃-C₁₅)-Cycloalkyl oder (C₆-C₁₄)-Aryl, wobei diese substituiert oder unsubstituiert sind,
R₇ = (C₁-C₁₅)-Alkyl, (C₃-C₁₅)-Cycloalkyl oder (C₆-C₁₄)-Aryl, wobei diese substituiert oder unsubstituiert sind, und
X = O, S
**dadurch gekennzeichnet,**
**dass** 3,5-disubstituierte 4-Hydroxybenzaldehyde 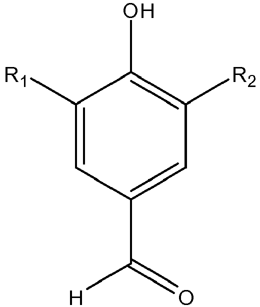 wobei R₁ und R₂ dieselbe Bedeutung haben wie oben,
mit Orthoformiaten der Formel 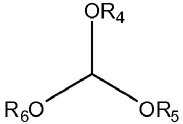 mit
R₄, R₅, R₆ = unabhängig voneinander (C₁-C₁₅)-Alkyl, (C₃-C₁₅)-Cycloalkyl oder (C₆-C₁₄)-Aryl, wobei diese substituiert oder unsubstituiert sind,
und Alkoholen und/oder Thioalkoholen der Formel 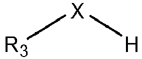 mit
R₃ = (C₁-C₁₅)-Alkyl, (C₃-C₁₅)-Cycloalkyl oder (C₆-C₁₄)-Aryl, wobei diese substituiert oder unsubstituiert sind, und
X = O, S,
in Gegenwart eines Katalysators, ausgewählt aus freien oder festphasengebundenen organischen Sulfonsäuren, Schwefelsäure, Hydrogensulfate, organischen oder anorganischen Phosphor-haltigen Säuren, deren Dihydrogen- und Hydrogensalze, sowie rauchender Salpetersäure und/oder Borsäure, zum entsprechenden Acetal umgesetzt wird und anschließend eine Eliminierung des Alkohols oder Thiols aus dem entsprechenden Acetal zum substituierten 1,4-Chinonmethid der Formel (I) erfolgt.

2. Verfahren gemäß Anspruch 1,
**dadurch gekennzeichnet, dass** als Katalysator freie oder festphasengebundene organischen Sulfonsäuren, Schwefelsäure und/oder Hydrogensulfate eingesetzt werden.

3. Verfahren gemäß Anspruch 1 oder 2,
**dadurch gekennzeichnet,**
**dass** das molare Verhältnis des 3,5-disubstituierten 4-Hydroxybenzaldehyds zum Katalysator von 1 : 0,001 bis 1 : 0,1 beträgt.

4. Verfahren gemäß zumindest einem der Ansprüche 1 bis 3,
**dadurch gekennzeichnet,**
**dass** das molare Verhältnis des 3,5-disubstituierten 4-Hydroxybenzaldehyds zum Orthoformiat von 1 : 1 bis 1 : 2 ist.

5. Verfahren gemäß zumindest einem der Ansprüche 1 bis 4,
**dadurch gekennzeichnet,**
**dass** das acetalhaltige Reaktionsgemisch auf mindestens 100°C erhitzt wird, wobei der freiwerdende (Thio-)Alkohol unmittelbar nach seinem Entstehen mittels chemischer und/oder physikalischen Methoden aus dem Reaktionsgemisch entfernt wird.

6. Verfahren gemäß Anspruch 5,
**dadurch gekennzeichnet,**
**dass** das acetalhaltige Reaktionsgemisch auf mindestens 100°C erhitzt, wobei zusätzliches Lösemittel (C) kontinuierlich zudosiert wird, während gleichzeitig der freiwerdende Alkohol und/oder Thioalkohol zusammen mit dem zusätzlichen Lösemittel (C) aus dem Reaktionsgemisch entfernt wird.

7. Verfahren zur Herstellung von substituierten 1,4-Chinonmethiden der Formel 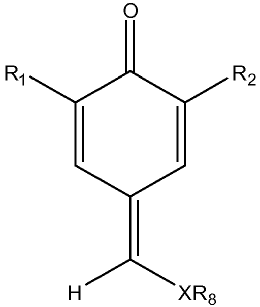 mit
R₁, R₂ = unabhängig voneinander Wasserstoff, (C₁-C₁₅)-Alkyl, (C₃-C₁₅)-Cycloalkyl oder (C₆-C₁₄)-Aryl, wobei diese substituiert oder unsubstituiert sind,
R₈ = (C₃-C₁₅)-Alkyl, (C₃-C₁₅)-Cycloalkyl oder (C₆-C₁₄)-Aryl, wobei diese substituiert oder unsubstituiert sind, und
X = O, S,
**dadurch gekennzeichnet,**
**dass** ein substituiertes 1,4-Chinonmethid der Formel 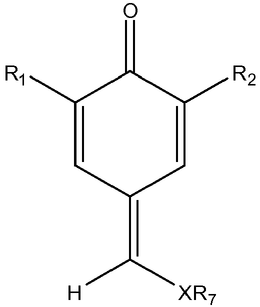 mit
R₇ = unsubstituierte (C₁-C₂)-Alkylgruppe und
X = O,
wobei R₁ und R₂ dieselbe Bedeutung wie oben haben, in Gegenwart eines Alkohols bzw. Thioalkohols der Formel 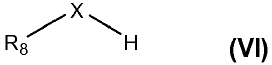 mit
X = O, S,
wobei R₈ dieselbe Bedeutung wie oben hat,
umgesetzt wird.

8. Verfahren gemäß Anspruch 7,
**dadurch gekennzeichnet,**
**dass** das eingesetzte substituierte 1,4-Chinonmethide mit R₇ = unsubstituierte (C₁-C₂)-Alkylgruppe und X = O mittels eines Verfahrens gemäß zumindest einem der Ansprüche 1 bis 6 hergestellt wird.

9. Verfahren gemäß Anspruch 1 zur Herstellung von 3,5-disubstituierten 4-Hydroxybenzaldehyds der Formel 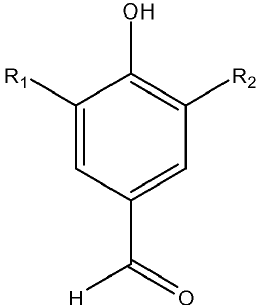 mit
R₁, R₂ = unabhängig voneinander Wasserstoff, (C₁-C₁₅)-Alkyl, (C₃-C₁₅)-Cycloalkyl oder (C₆-C₁₄)-Aryl, wobei diese substituiert oder unsubstituiert sind,
**dadurch gekennzeichnet, dass** 2,6-disubstituiertes Phenol gemäß der Formel 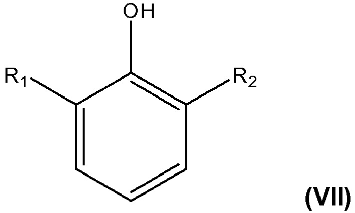 wobei R₁ und R₂ dieselbe Bedeutung haben wie oben,
mit Urotropin in einem Lösemittelgemisch bestehend aus Eisessig und Wasser bei Temperaturen, die während der gesamten Reaktionszeit mindestens 2°C unterhalb der Rückflusstemperatur liegen, umgesetzt wird und das molare Verhältnis des 2,6-disubstituierten Phenols zum Urotropin von 1 : 1 bis 1 : 0,8 ist.

## Claims

1. A process for preparing substituted 1,4-quinone methides of the formula 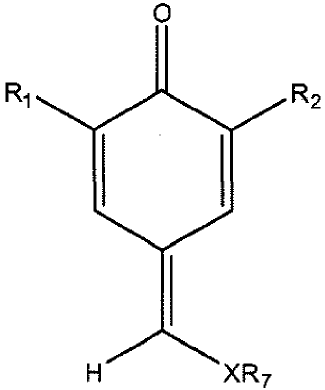 where
R₁, R₂ = each independently hydrogen, (C₁-C₁₅)-alkyl, (C₃-C₁₅) -cycloalkyl or (C₆-C₁₄)-aryl, which are unsubstitued or substituted,
R₇ = (C₁-C₁₅)-alkyl, (C₃-C₁₅) -cycloalkyl or (C₆-C₁₄)-aryl, which are substituted or unsubstituted, and
X = O, S,
**characterized in that**
3,5-disubstituted 4-hydroxybenzaldehydes 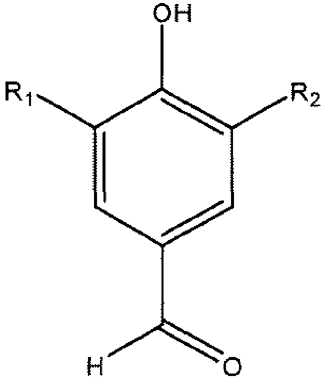 where R₁ and R₂ are each as defined above
are reacted with orthoformates of the formula 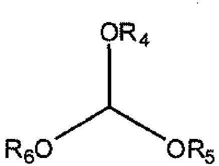 where
R₄, R₅, R₆ = each independently (C₁-C₁₅)-alkyl, (C₃-C₁₅) -cycloalkyl or (C₆-C₁₄) - aryl, which are substitued or unsubstituted,
and alcohols and/or thioalcohols of the formula 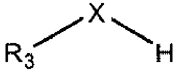 where
R₃ = (C₁-C₁₅)-alkyl, (C₃-C₁₅)-cycloalkyl or (C₆-C₁₄)-aryl, which are substituted or unsubstituted, and
X = O, S,
in the presence of a catalyst selected from free or solid-phase-bound organic sulphonic acids, sulphuric acid, hydrogensulphates, organic or inorganic phosphorus acids, the dihydrogen and hydrogen salts thereof, and fuming nitric acid and/or boric acid, to give the corresponding acetal and then the alcohol or thiol is eliminated from the corresponding acetal to give the substituted 1,4-quinone methide of the formula **(I).**

2. A process according to claim 1,
**characterized in that** the catalysts used are free or solid-phase-bound organic sulphonic acids, sulphuric acid and/or hydrogensulphates.

3. A process according to claim 1 or 2,
**characterized in that**
the molar ratio of the 3,5-disubstituted 4-hydroxybenzaldehyde to the catalyst is from 1:0.001 to 1:0.1.

4. A process according to at least one of claims 1 to 3,
**characterized in that**
the molar ratio of the 3,5-disubstituted 4-hydroxybenzaldehyde to the orthoformate is from 1:1 to 1:2.

5. A process according to at least one of claims 1 to 4,
**characterized in that**
the acetal-containing reaction mixture is heated to at least 100°C, the (thio)alcohol released being removed from the reaction mixture by means of chemical and/or physical methods immediately after formation thereof.

6. A process according to claim 5,
**characterized in that**
the acetal-containing reaction mixture is heated to at least 100°C, additional solvent (C) being metered in continuously while the alcohol and/or thioalcohol released is simultaneously removed from the reaction mixture together with the additional solvent (C).

7. A process for preparing substituted 1,4-quinone methides of the formula 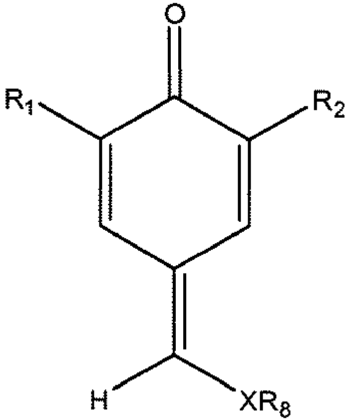 where
R₁, R₂ = each independently hydrogen, (C₁-C₁₅)-alkyl, (C₃-C₁₅)-cycloalkyl or (C₆-C₁₄)-aryl, which are unsubstitued or substituted,
R₈ = (C₃-C₁₅)-alkyl, (C₃-C₁₅)-cycloalkyl or (C₆-C₁₄)-aryl, which are substituted or unsubstituted, and
X = O, S,
**characterized in that**
a substituted 1,4-quinone methide of the formula 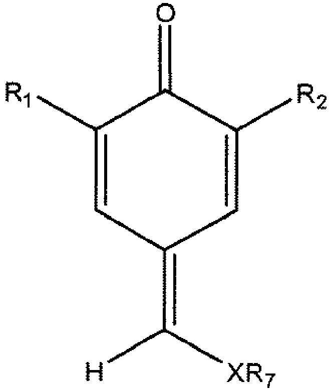 where
R₇ = unsubstituted (C₁-C₂)-alkyl group and
X = O,
where R₁ and R₂ are each as defined above, is reacted in the presence of an alcohol or thioalcohol of the formula 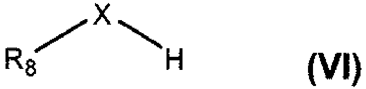 where
X = 0, S,
where R₈ is as defined above.

8. A process according to claim 7,
**characterized in that**
the substituted 1,4-quinone methides where R₇ = unsubstituted (C₁-C₂)-alkyl group and X = 0 used are prepared by means of a process according to at least one of claims 1 to 6.

9. A process according to claim 1 for preparing 3,5-disubstituted 4-hydroxybenzaldehyde of the formula 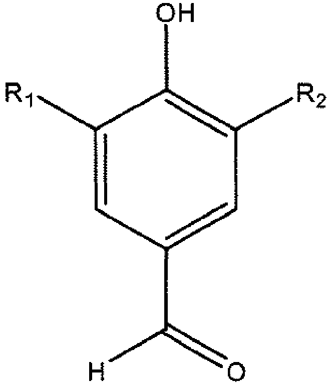 where
R₁, R₂ = each independently hydrogen, (C₁-C₁₅)-alkyl, (C₃-C₁₅)-cycloalkyl or (C₆-C₁₄)-aryl, which are substitued or unsubstituted,
**characterized in that**
2,6-disubstituted phenol of the formula 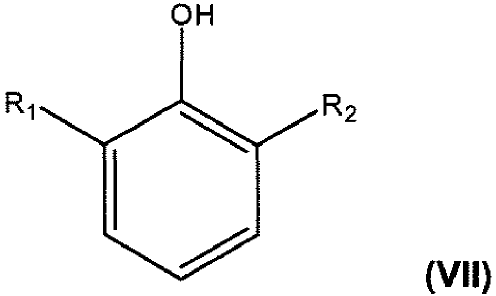 where R₁ and R₂ are each as defined above
is reacted with urotropin in a solvent mixture consisting of glacial acetic acid and water at temperatures which are at least 2°C below the reflux temperature over the entire reaction time and the molar ratio of the 2,6-disubstituted phenol to the urotropin is from 1:1 to 1:0.8.

## Revendications

1. Procédé de fabrication de 1,4-quinone-méthides substitués de formule 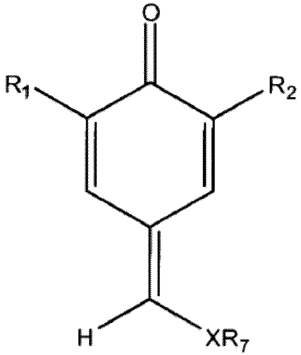 avec
R₁, R₂ = indépendamment l'un de l'autre, hydrogène, alkyle en (C₁-C₁₅), cycloalkyle en (C₃-C₁₅) ou aryle en (C₆-C₁₄), ceux-ci étant substitués ou non substitués,
R₇ = alkyle en (C₁-C₁₅), cycloalkyle en (C₃-C₁₅) ou aryle en (C₆-C₁₄), ceux-ci étant substitués ou non substitués,
et
X = 0, S,
**caractérisé en ce que**
des 4-hydroxybenzaldéhydes 3,5-disubstitués 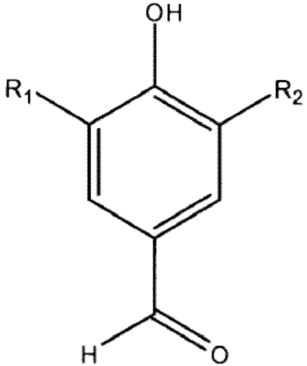 R₁ et R₂ ayant la même signification que précédemment, sont mis en réaction avec des orthoformiates de formule 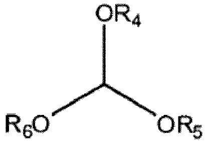 avec R₄, R₅, R₆ = indépendamment les uns des autres, alkyle en (C₁-C₁₅), cycloalkyle en (C₃-C₁₅) ou aryle en (C₆-C₁₄), ceux-ci étant substitués ou non substitués,
et des alcools et/ou des thioalcools de formule 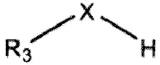
avec R₃ = alkyle en (C₁-C₁₅), cycloalkyle en (C₃-C₁₅) ou aryle en (C₆-C₁₄), ceux-ci étant substitués ou non substitués, et
X = 0, S,
en présence d'un catalyseur, choisi parmi les acides sulfoniques organiques libres ou liés à une phase solide, l'acide sulfurique, les hydrogénosulfates, les acides organiques ou inorganiques contenant du phosphore, leurs sels dihydrogène ou hydrogène, ainsi que l'acide nitrique fumant et/ou l'acide borique, pour former l'acétal correspondant, puis une élimination de l'alcool ou du thiol de l'acétal correspondant a lieu pour former le 1,4-quinone-méthide substitué de formule (I).

2. Procédé selon la revendication 1, **caractérisé en ce que** des acides sulfoniques organiques libres ou liés à une phase solide, de l'acide sulfurique et/ou des hydrogénosulfates sont utilisés en tant que catalyseur.

3. Procédé selon la revendication 1 ou 2, **caractérisé en ce que** le rapport molaire entre le 4-hydroxybenzaldéhyde 3,5-disubstitué et le catalyseur est de 1:0,001 à 1:0,1.

4. Procédé selon au moins l'une quelconque des revendications 1 à 3, **caractérisé en ce que** le rapport molaire entre le 4-hydroxybenzaldéhyde 3,5-disubstitué et l'orthoformiate est de 1:1 à 1:2.

5. Procédé selon au moins l'une quelconque des revendications 1 à 4, **caractérisé en ce que** le mélange réactionnel contenant un acétal est porté à au moins 100 °C, le (thio)alcool libéré étant éliminé du mélange réactionnel immédiatement après sa formation au moyen de méthodes chimiques et/ou physiques.

6. Procédé selon la revendication 5, **caractérisé en ce que** le mélange réactionnel contenant un acétal est porté à au moins 100 °C, un solvant supplémentaire (C) étant ajouté en continu, tandis que l'alcool et/ou le thioalcool libéré est simultanément éliminé du mélange réactionnel conjointement avec le solvant supplémentaire (C).

7. Procédé de fabrication de 1,4-quinone-méthides substitués de formule 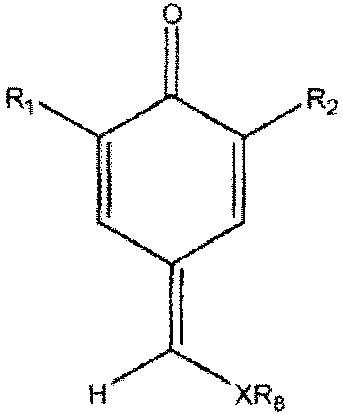 avec
R₁, R₂ = indépendamment l'un de l'autre, hydrogène, alkyle en (C₁-C₁₅), cycloalkyle en (C₃-C₁₅) ou aryle en (C₆-C₁₄), ceux-ci étant substitués ou non substitués,
R₈ = alkyle en (C₁-C₁₅), cycloalkyle en (C₃-C₁₅) ou aryle en (C₆-C₁₄), ceux-ci étant substitués ou non substitués,
et
X = O, S
**caractérisé en ce que**
un 1,4-quinone-méthide substitué de formule 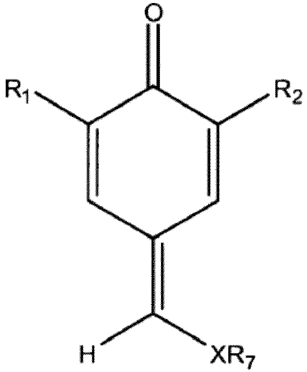
avec R₇ = groupe alkyle en (C₁-C₂) non substitué, et
X = O,
R₁ et R₂ ayant la même signification que précédemment, est mis en réaction en présence d'un alcool ou thioalcool de formule 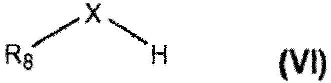
avec X = 0, S,
R₈ ayant la même signification que précédemment.

8. Procédé selon la revendication 7, **caractérisé en ce que** le 1,4-quinone-méthide substitué utilisé avec R₇ = groupe alkyle en (C₁-C₂) non substitué et X = 0 est fabriqué par un procédé selon au moins l'une quelconque des revendications 1 à 6.

9. Procédé selon la revendication 1, pour la fabrication de 4-hydroxybenzaldéhyde 3,5-disubstitué de formule 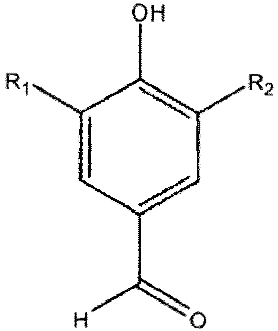 avec R₁, R₂ = indépendamment l'un de l'autre, hydrogène, alkyle en (C₁-C₁₅), cycloalkyle en (C₃-C₁₅) ou aryle en (C₆-C₁₄), ceux-ci étant substitués ou non substitués,
**caractérisé en ce que**
qu'un phénol 2,6-disubstitué de formule 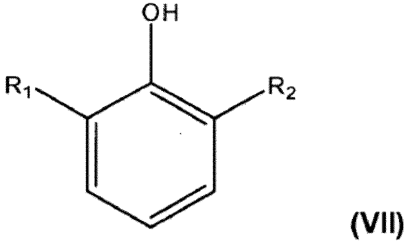 R₁ et R₂ ayant la même signification que précédemment, est mis en réaction avec de l'urotropine dans un mélange de solvants constitué par de l'acide acétique glacial et de l'eau à des températures qui se situent pendant l'ensemble de la durée de réaction au moins 2 °C en-dessous de la température de reflux, le rapport molaire entre le phénol 2,6-disubstitué et l'urotropine étant de 1:1 à 1:0,8.
